# EUROPEAN PATENT APPLICATION

(11) **EP 2 535 411 A1**
(43) Date of publication of application: **19.12.2012**
(21) Application number: 10845835.7
(22) Date of filing: 12.02.2010
(51) Int. Cl.: C12N 15/11, C12Q 1/68

(54) **PROBE FOR HPV GENOTYPE DIAGNOSIS AND ANALYSIS METHOD THEREOF**

(71) Applicant: M&D.Inc., Gangwon-do 220-710 (KR)
(72) Inventor: CHO, Sang-Nae, Seoul 158-056 (KR); KIM, Tae-Woo, Wonju-si Gangwon-do 220-750 (KR); PARK, Kwang-Hwa, Wonju-si Gangwon-do 220-981 (KR); LEE, Hye-Young, Wonju-si Gangwon-do 220-150 (KR); LEE, Dong-Sup, Wonju-si Gangwon-do 220-981 (KR); KANG, Mi-Ran, Wonju-si Gangwon-do 220-750 (KR); WANG, Hye-Young, Gunsan-si Jeollabuk-do 573-754 (KR); KIM, Sung-hyun, Daejeon 302-242 (KR)
(74) Representative: Richter Werdermann Gerbaulet Hofmann
(86) International application number: PCT/KR2010/000914
(87) International publication number: WO 2011/099664

(57) **Abstract**

The present invention relates to a probe for diagnosing HPV genotype and to a method of analyzing the same.

## Description

### [Technical Field]

The present invention relates to a probe for diagnosing HPV genotype and a method of analyzing the same.

### [Background Art]

It is known that HR (High Risk)-HPV DNA is present at 99.7% of patients suffered with uterine cervical cancer all over the world and a continuous existence of HR-HPV infection causes metastasis to invasive uterine cervical cancer and cervical intraepithelical neoplasia. The new 6000 patients of uterine cervical cancer have appeared in Korea and half a million patients of uterine cervical cancer has appeared every year all over the world. 50% of them can have fatal consequences. The presence or absence of HPV DNA is being used for an early diagnosis of uterine cancer as Human Papilloma Virus (HPV) becomes known to be an important cause for the process of the uterine cervical cancer (Bernard, H. U., I. E. Calleja-Macias, and S. T. Dunn. 2006. Int J Cancer 118:1071-6). They are known that HPV is DNA virus having approximately 7.9 kb double helix structure and belongs to papovavirus, and there are at least 100 subtypes of HPV and 40 subtypes of them are transmitted via genital organs. In addition, HPV virus has 10 viral proteins, which expresse L1 and L2 that are two early gene products involved in a synthesis of structural protein consisting capsid of virus and E1, E2, E3, E4, E5, E6, E7, and E8 that are eight late gene products regulating protein synthesis involved in a replication of virus determining an action and latent of virus infection. Especially, E6 and E7 encode precancer protein and are known to be parts involved in cancer (Sedman, S. A., et. al., 1991. J Virol 65:4860-6). According to many reports, it is known that HPV virus causes uterine cervical cancer in women and is closely connected with various malignant tumors (Godfroid, E., et. al., 1998. J Virol Methods 75:69-81).

Uterine cervical cancer of women shows the second highest attack rate after breast cancer all over the world and at least 1,000 patients are killed in Korea. Hence it is very important to strive for an early diagnosis of uterine cervical cancer (Wui, J. H., et. al., 2006. Journal of Gynecologic oncology and Coloscopy 17:39-4711).

At least 100 HPV genotypes are known now (Likes, W. M., and J. Itano. 2003. Clin J Oncol Nurs 7:271-6). Among these, approximately 30 HPV genotypes that can lead to diseases to people are known. The genotypes are classified into a high risk group (16, 18, 31, 33, 35, 39, 45, 51, 52, 56, 58, 59, 68, 73, and 82), a low risk group (6, 11, 40, 42, 43, 44, 54, 61, 70, 72, and 81), and a potential risk group (34, 57, and 83) (Munoz, N., et. al., 2003. N Engl J Med 348:518-27). Biological diversity of HPV infection is recognized by confirming specific genotype-HPV having each of these genotypes according to lesion site and the degree of the progress of lesion.

The test most often used in a diagnosis to know whether the HPV is transmitted is Papnicolaou Smear (Pap) test that is now used in a histological diagnosis of uterine cervical cancer, but has disadvantages in that the test should considerably depend on an experience and skill level of tester and thus is generally less accurate (Kurman, R. J., D. E. Henson, A. L. Herbst, K. L. Noller, and M. H. Schiffman. 1994. JAMA 271:1866-9). Furthermore, a test using a colposcope can obtain the result accurately than Papnicolaou Smear test, but there are disadvantages in that it requires an experienced technician and expensive equipment, and is not able to distinguish HPV genotypes. In addition, there is a disadvantage that HPV genotypes having different degree of risk to malignant alteration can be not classified via both of the tests.

Studies on the tests for diagnosing a precancer step of uterine cervical cancer and confirming genotype through a HPV-specific DNA amplification by using PCR, HC, DNA chip, and the like, are being carried out to compensate the above disadvantages.

### [Disclosure]

### [Technical Problem]

An object of the present invention is to provide a probe with good specificity and sensitivity to HPV as compared with DNA chip in the related art, so as to solve the problems and meet the needs as mentioned above.

Another object of the present invention is to provide a method of analyzing HPV genotype by using the probe.

### [Technical Solution]

In order to achieve the above objects, an exemplary embodiment of the present invention provides an oligonucleotide probe for diagnosing at least one HPV selected from the group consisting of base sequences of Sequence Nos. 5 to 28.

In addition, an exemplary embodiment of the present invention provides a kit for diagnosing HPV, including the probe according to the present invention and a primer.

For the present invention, the primer is preferably disclosed in Sequence Nos. 1 to 4, but the present invention is not limited thereto.

For an exemplary embodiment of the present invention, preferably, the kit further includes a marker for detecting PCR product.

In addition, an exemplary embodiment of the present invention provides a method of confirming HPV genotype, in which the method includes:
a) attaching the probe according to the present invention to a solid support;
b) hybridizing HPV PCR product amplified to the probe; and
c) detecting the hybridized product.

For the present invention, the solid support preferably includes a membrane, a slide, or a well plate, but the present invention is not limited thereto.

For an exemplary embodiment of the present invention, the step of detecting is preferably performed by using a luminous or color reaction.

The present invention is performed by extracting nucleic acid from a clinical specimen and then amplifying the nucleic acid using PCR. The methods for extracting nucleic acid from cell and for performing PCR are performed by using the known method in the related art, such as the method as disclosed in Molecular cloning (1989) by Sambrook, *et al.*

A method of amplifying nucleic acid according to the present invention uses a primer. Generally, the primer is preferably oligonucleotide with the length of approximately 10 to 25 bp, but the oligonucleotide with a little longer than that of the oligonucleotide is also possible. The primer is preferably a single strand, but it may be presented in a type of double or single strand. The specific primer used in the present invention will be described in the following Example.

The primer according to the present invention is prepared by using a general method for synthesizing oligonucleotide and the above method for synthesizing is disclosed in US Patent Nos. 4,659,774, 4,816,571, 5,141,813, 5,264,566, 4,959,463, 5,428,148, 5,554,744, 5,574,146, 5,602,244, and the like.

The sequences as the primer according to the present invention may be used for forming double helix selectively with complementary sequences, such as DNA derived from sample. A person of ordinary skill in the art can change the conditions of hybridization in order to change selectivity of a primer to a target sequence.

In some Examples, the nucleic acid of limited sequence according to the present invention may be selected by binding with a proper mean, such as a marker in order to determine hybridization. The proper marker may include various markers including fluorescence, a radioactive isotope, enzyme, or other ligand.

A specific gene product that is present in a given cell sample can be amplified by using processes depending on various templates. One of the most known methods of amplifying is a polymerase chain reaction (PCR). The method is disclosed in US patent Nos. 4,683,202, 4,800,159, and the like.

Generally, an amplified product is possible to be isolated from templates, excess primers, or other amplified products in one step or another step. For example, the amplified product can be isolated by using the standard method as disclosed in Molecular cloning (1989) by Sambrook, *et al.* through agarose, agarose-acrylamide, or PAGE.

In addition, a chromatograph, such as absorption, a distribution, an ionexchange, and the like can be adopted as an effective isolation method. The methods of isolating may be adopted to be functioned in a clinical setting in order to process a large quantity of sample. In addition, there are fluorometric microvolume assay technique (FMAT), chemiluminescence, sequence detection system (Applied Biosystems), mass spectroscopy, and the like as a novel method to isolate or detect thousands of samples at a time.

The nucleic acid according to the present invention may be detected and then quantified. For an exemplary embodiment of the present invention, the detection may be performed by using a visual mean. A traditional visual method is to dye gel with ethidium bromide and then visualize bands under UV light. In addition, when an amplified product is nucleotide marked with radiation or fluorescence, the isolating amplified product is subjected to be a fluorescence detection or radiation scintigraphy of inserted radiation isotope marker.

The present invention includes a kit for performing the above method. For non-limited Example, the kit according to the present invention may include primers, enzymes for amplification, and additive reagents. Accordingly, the kits according to the present invention may include at least one reagent in a proper container. The kits may also include the reagents for isolating nucleic acid or purifying the amplified product. Constituents of the kits may be packaged in a freeze-drying type or aqueous medium and the proper container of the kit may include at least one vial, test tube, a flask, a bottle, and the like, that can receive the constituents. When the kit may include at least one constituent, the kit may include other additive container, such as second one, third one, and the like, in which the additive constituents may be located separately. However, the combination of various constituents may be also included in one container.

The nested PCR method used for the present invention relates to a general Two-tube nested PCR. In terms of 'Two-tube nested PCR,' when the amount of DNA in the general sample is very limited thereby not detecting using only one PCR, a second PCR is performed by using more inner primer. Four primers should be attached to a desired part to amplify well, and first PCR should be performed by using outer primers. When the second amplification is performed by using the resulting product, there are advantages in that since new reagents are added again, the reaction may be effectively performed and since one pair of primers is used for relatively small number of times, it can increase specificity and can have excellent sensitivity as compared with PCR in the related art. However, there is also a disadvantage in that contamination may be caused because of two amplification processes by changing a tube. In order to solve the disadvantage, two pairs of primers are added to one tube and then one-tube nested PCR is performed. Accordingly, it has been seen that the problem of the contamination can be decreased and also it can have good sensitivity as compared with the two-tube PCR in the related art (see Example 8).

A method of confirming HPV genotype according to the present invention includes amplifying HPV L1 gene using PCR, and performing a reverse blot hybridization of the amplified DNA using ECL-Southern hybridization on a membrane attached with a probe that can be specifically attached to each HPV type. A final object of the present invention is to develop a probe with good sensitivity and more specificity as compared with DNA chip in the related art and also to develop more economical method.

Especially, the present invention is to provide a novel method of diagnosing HPV by confirming a rate of detection of HPV using one-tube nested PCR that amplifies in one tube with two pairs of primers, and then comparing the result of the PCR with other diagnosis methods.

As shown in the construction and figures in the present invention, a method of confirming HPV genotype according to the present invention includes amplifying HPV L1 gene using PCR, and performing a reverse blot hybridization of the amplified DNA using ECL-Southern hybridization on a membrane attached with a probe that can be specifically attached to each HPV type, in which the method is to develop a probe with good sensitivity and more specificity as compared with DNA chip in the related art and also to develop more economical method.

### [Description of Drawings]

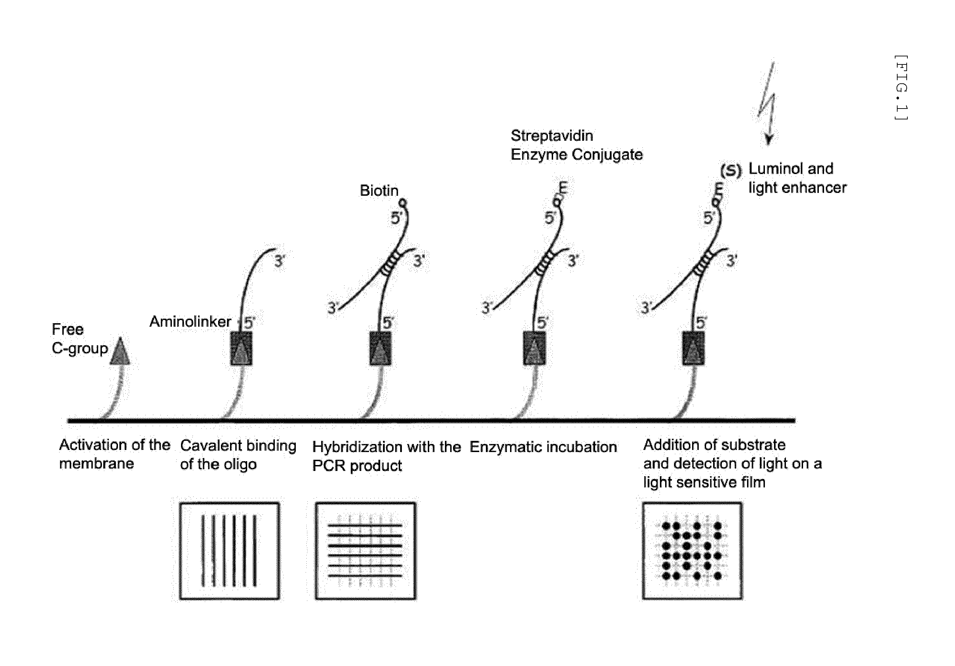

FIG. 1 is an explanation diagram illustrating a basic principle of reverse blot hybridization as mentioned in the present invention. That is, the reverse blot hybridization includes ① binding a probe marking amine group to a membrane, ② amplifying target gene with a primer labeled with biotin and then reacting with the probe bond to the membrane, ③ reacting with streptavidin-conjugated alkaline phosphatase, and then ④ detecting the binding of target gene amplified and the probe by treating a substrate of the alkaline phosphatase.

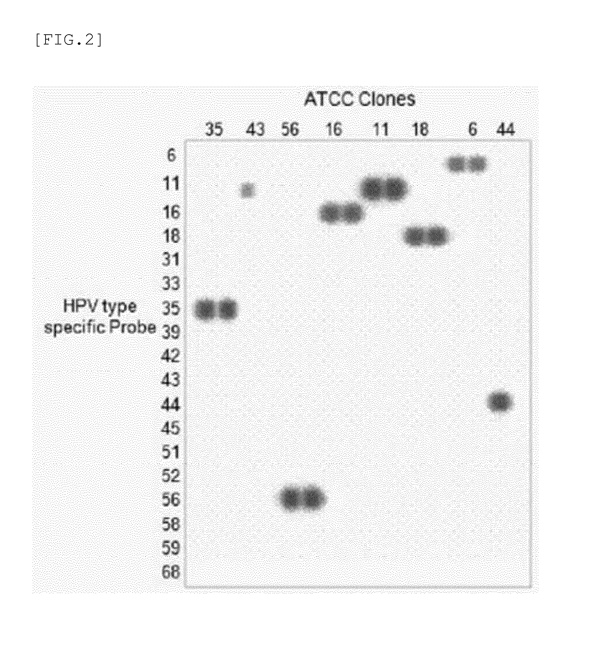

FIG. 2 is a photograph illustrating detection through Luminous after performing the reverse blot hybridization reaction using the probe bind to the membrane and HPV L1 genes amplified via PCR from HPV type 35, 43, 56, 16, and 18 clones belonging to a high risk group and HPV type 6, 11, and 44 clones belonging to a low risk group among obtained ATCC clones.

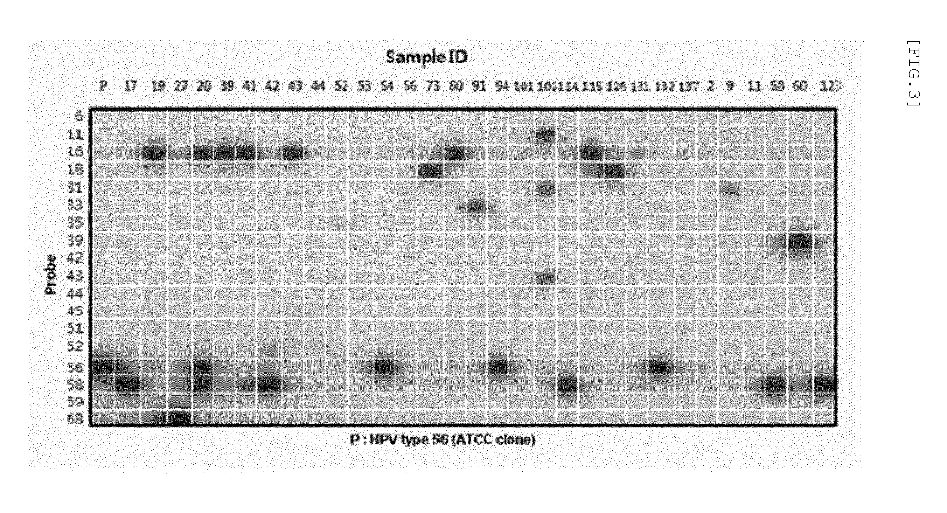

FIG. 3 is a photograph illustrating REBA result of Capture^{®}2 High-Risk HPV DNA Test™ (HC2) positive clinical specimen. In other words, the photograph illustrates that 31 clinical specimens were PCR positive among total 44 clinical specimens from Digene (HC2) positive specimens and it has been seen from the result after performing the reverse blot hybridization luminous experiment (REBA) of the 31 clinical specimens that total 26 clinical specimens were HPV type, i.e., 19 clinical specimens were single type of 16, 18, 31, 33, 35, 39, 56, and 58; 4 clinical specimens were double type of 16/18, 16/58, 52/58, and 59/68; and 3 clinical specimens were triple type of 16/51/58, 16/56/58, and 16/51/58.

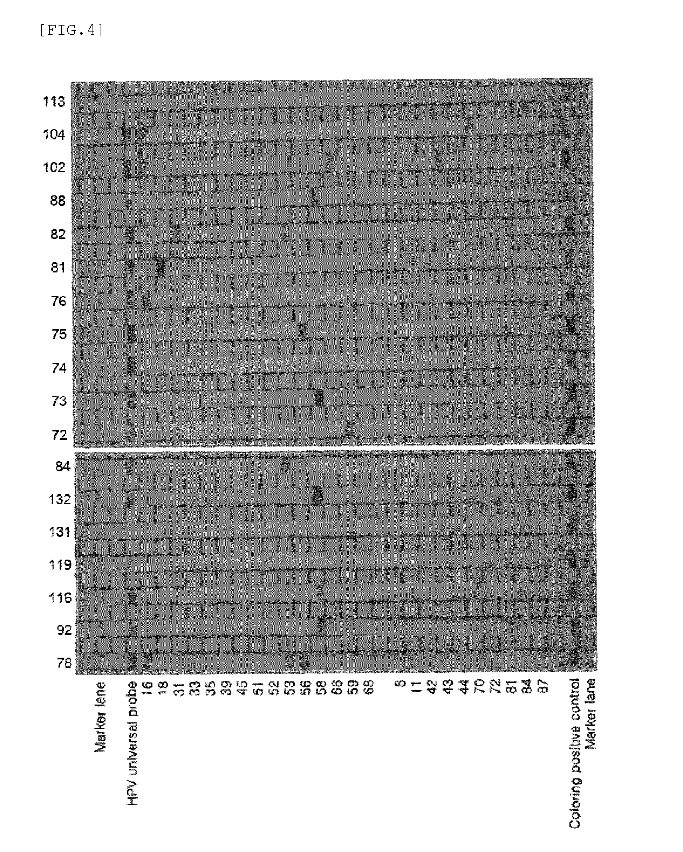

FIG. 4 is a photograph illustrating REBA result of clinical samples (Color test). In other words, the photograph illustrates that it has been seen from the results after performing a color test using membrane strip with 18 clinical samples that total 17 clinical samples were isolated in each type of HPV types, i.e., 12 clinical specimens were single type of 16, 18, 53, 56, 58, 59, 70, and 81; 3 clinical specimens were double type of 58/70, 31/53, and 66/43; and 1 clinical specimen was triple type of 16/53/56; and 1 clinical specimen was HPV positive like 113 sample; and 113 sample could not be subjected to be performed using PCR and thus there were no bands in REBA color test.

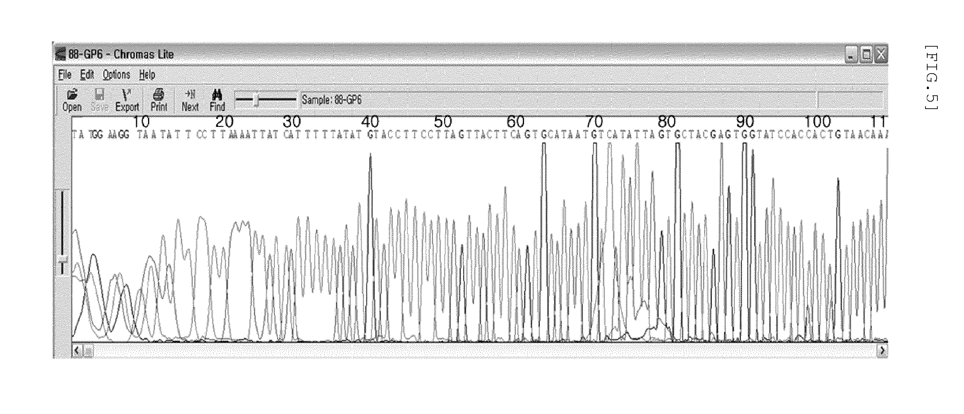

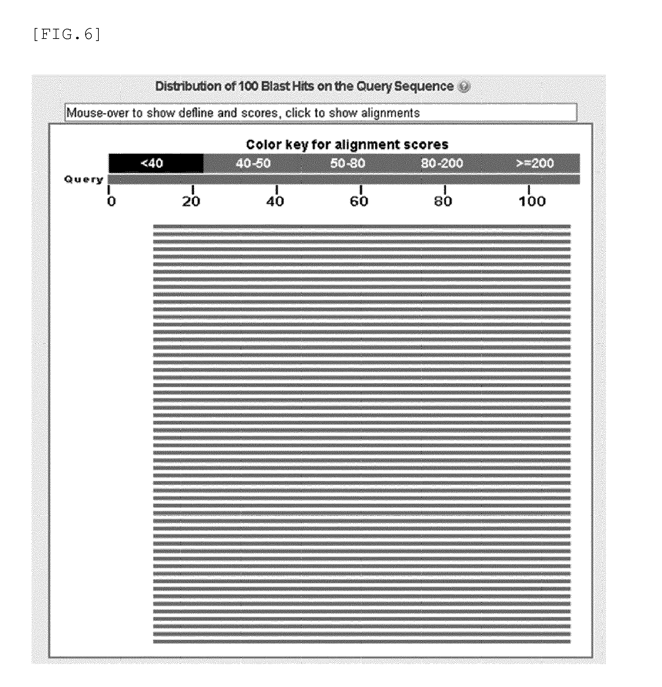

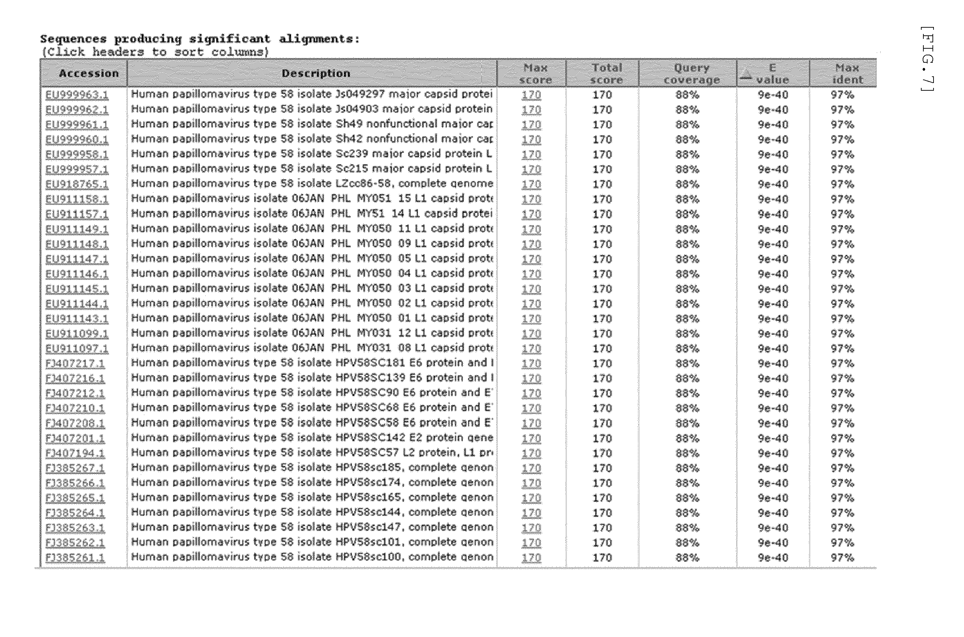

FIGS. 5 to 7 are diagrams illustrating the result of sequencing to REBA results of clinical samples (Color test - 88). In other words, FIG. 5 is a photograph illustrating the sequencing result by listing each of base sequences using chromas program after sequencing 88-clinical sample that shows a color from the color test of FIG. 4, and FIGS. 6 and 7 are photographs illustrating the result from blast search in http://blast.ncbi.nlm.nih.gov/Blast.cgi site with base sequence from FIG. 5.

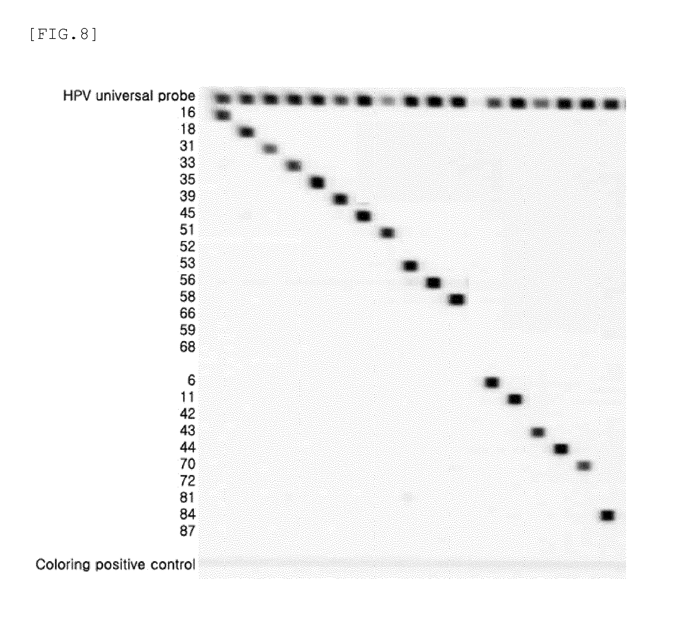

FIG. 8 is a photograph illustrating REBA result of Plasmid DNA clone (Luminous Experiment). In other words, the photograph was obtained by cloning each type of clinical samples obtained in order to use it as a positive control, and then performing REBA (Luminous Experiment) with the plasmid DNA clone obtained from the above cloning.

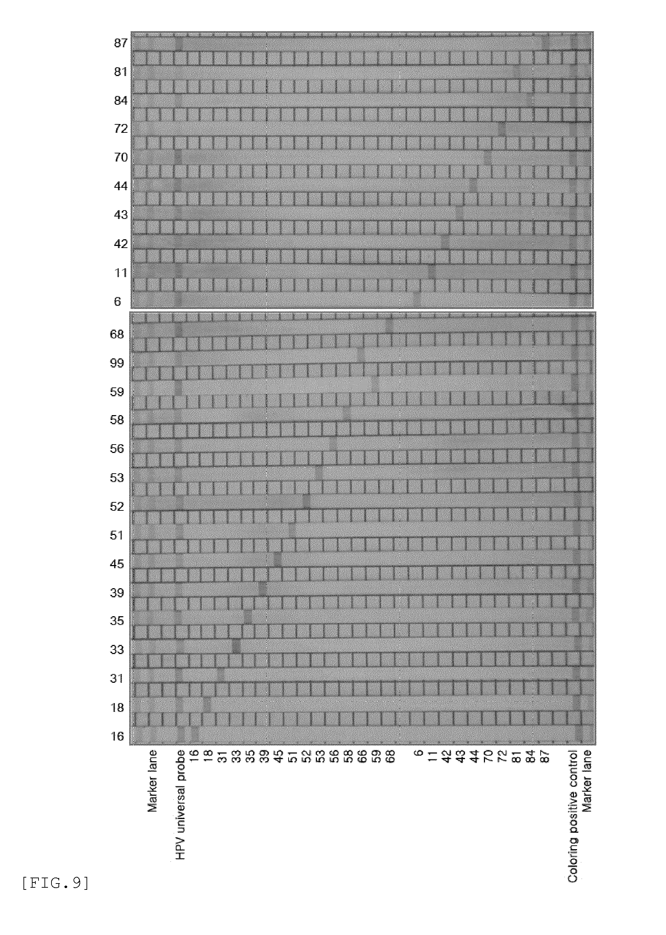

FIG. 9 is a photograph illustrating REBA result of Plasmid DNA clone (Color Test). In other words, the photograph was obtained by cloning each type of clinical samples obtained in order to use it as a positive control, and then performing REBA (Color Test) with the plasmid DNA clone obtained from the above cloning.

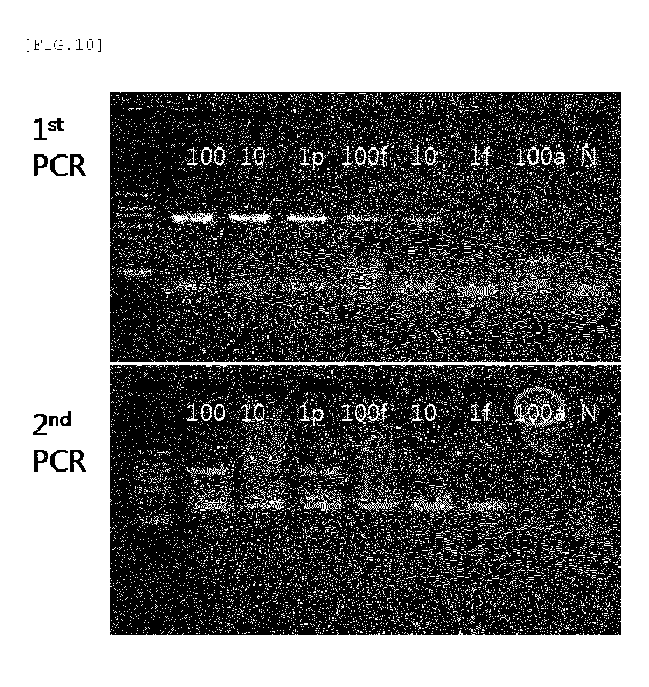

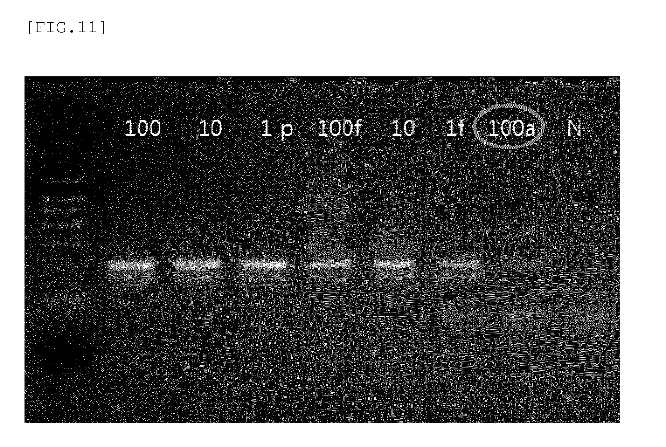

FIGS. 10 and 11 are photographs illustrating the results of one-tube nested PCR and two-tube nested PCR, respectively.

### [Best Mode]

Hereinafter, the present invention will be described in more detail through the following non-limited Examples. However, the following Examples are only for illustrating the present invention and the range of the present invention will not be limited to the following Examples.

### Example 1: Obtainment of HPV Positive Clone

The plasmids corresponding to eight HPV genotypes among HPV genotypes were obtained from ATCC. Especially, pHPV-16 (Cat. No. 45133) was used as a positive control for the whole experiments. Eight plasmids obtained from ATCC were HPV-6b (Cat. No. 45150), HPV-11 (Cat. No. 45151), HPV-18 (Cat. No. 45152), HPV-35 clone 2A (Cat. No. 40330), HPV-43 Clone 2A (Cat. No. 40338), HPV-44 Clone 2 (Cat. No. 40353), and HPV-56 Clone 2C (Cat. No. 40549). Since all of them were transformed into *E. coli,* plasmid DNAs were extracted after culturing (GeneAll, Seoul, Korea) and used as templates for a polymerase chain reaction (PCR).

### Example 2: Obtainment of HPV Clinical Specimen

Clinical specimens used for the present invention were obtained from 44 patients that were diagnosed with a high risk (HR) group among 137 patient already tested for Hybrid Capture 2 High-Risk HPV DNA Test™ (HC2, Digene, Gaithersburg, USA) in Gangseo MizMedi Hospital for 6 months from November, 2005, and then used as a experimental group of the present invention. In order to increase efficiency of PCR in the case of the clinical specimens, the same amount of 0.45 N HCl was added and mixed to neutralize the solution including DNA, and then used as a template. In addition, in order to verify reliability of the present invention, a high risk group, i.e., HPV type 16, 31, 33, 39, 51, 56, and 58 and a low risk group, i.e., HPV type 6 and 11, of which genotyping was completed by using sequencing, among the clinical specimens isolated from the patients of MezMedi hospital in Korea, were obtained. HPV type 42 and 45 were obtained from Biometrics; and the high risk group, i.e., HPV type 52, 53, 59, and 68 and the low risk group, i.e., HPV type 70, 72, 81, 84, and 87 were obtained from Chonnam national university hospital and Wonju Christian hospital.

### Example 3: PCR Reaction

PCR reaction was performed after confirming whether an inhibitor was in DNA product by first performing beta-globin PCR. A reaction condition was as follows: a denaturation at 94°C of a denaturation temperature for 5 minutes; a denaturation at 94°C for 30 seconds; an annealing at 55°C for 30 seconds; and an extension at 72°C for 30 seconds were repeated in total 35 times, and then finally, a final extension reaction was performed at 72°C for 7 minutes.

After the reaction, one-tube nested PCR was performed with DNA that was the product (positive specimen) amplified through PCR. 20 mM Tris-HCl (pH 8.0), 100 mM KCl, 0.1 mM EDTA, 1 mM DTT, 0.5% Tween-20, 0.5% Nonidet P-40, each of 10 mM dNTPs, 25mM MaCl₂, each of 10 pmole primers, and 1 unit *Taq* polymerase were used to be 20 µl of the total reaction amount in 200 µl tube for PCR.

A reaction condition was as follows: a pre-denaturation at 94°C of a denaturation temperature for 5 minutes, a denaturation at 94°C for 30 seconds, and an annealing at 55°C for 30 seconds were first reacted in 15 cycles; then again a denaturation at 94°C for 30 seconds and an annealing at 52°C for 30 seconds were reacted in 45 cycles; and then a final extension at 72°C for 10 minutes was reacted finally. At this time, the primer used was MY11/09-GP5-1/6+ to obtain 150 bp amplication product. A base sequence of each primer is shown in Table 1.

**[Table 1]**

| | **Sequence** |
|---|---|
| **Primer** | |
| MY11 (forward) | GCM CAG GGW CTA TAA YAA TGG |
| MY09 (reverse) | CGT CCM ARR GGA WAC TGA TC |
| GP5-1 (forward) | TTTGTTACWGTKGTRGATAC |
| GP6+ (reverse) | GAA AAA TAA ACT GTA AAT CAT ATT C |

Table 1 is a primer sequence. The sequences of MY11, MY09, GP5-1, and GP6+ in Table 1 are disclosed in Sequence Nos. 1 to 4, respectively.

**[Table 2]**

| | | |
|---|---|---|
| Predenaturation | 94°C 5 min | |
| Denaturation | 94°C 30 sec | |
| Annealing | 55°C 30 sec | 15 cycles |
| Denaturation | 94°C 30 sec | |
| Annealing | 52°C 30 sec | 45 cycles |
| Final elongation | 72°C 10 min 1 cycles | |
| | 4°C Storage | |

Table 2 is a PCR condition.

### Example 4: Preparation of Membrane Attached with Probe and Oligonucleotide Used as Probe

A base sequence of oligonucleotide of HPV type specific probe is in Table 3. We asked Bioneer (Korea) to prepare oligonucleotide that has amine group at its 5'-terminal to have positive potential for binding with carboxyl group that has negative potential of the membrane, for using as a probe. In order to attach the probe prepared to the membrane, the probe was diluted with 0.5 M NaHCO₃ solution to be 100 pmole of the total probe concentration (the concentration was regulated per each type), the probe was loaded to each slot using Miniblotter-MN45 (Immunetics, Cambridge, MA) that was reacted with 1.6 g/10 ml EDAC for 10 minutes, and then reacted at room temperature for 1 hour. Since then, it was reacted with 100 mM NaOH for 9 minutes, and then finally reacted with 100 ml 2X SSPE/0.1% (w/v) SDS solution at 60°C for 5 minutes to remove non-bond probe.

**[Table 3]**

| | HPV type (Seq. No.) | Probe Sequence | | HPV type (Seq. No.) | Probe Sequence |
|---|---|---|---|---|---|
| HR | 16(5) | CATTATGTGCTGCCATATC | LR | 6(19) | TCCGTAACTACATCTTCCA |
| | 18(6) | TGCTTCTACACAGTCTCCT I | | 11(20) | TCTGTGTCTAAATCTGCTAC |
| | 31(7) | GCAATTGCAAACAGTGATAC | | 12(21) | TGGTGATACATATACAGCTC |
| | 33(8) | TGCACACAAGTAACTAGTGA | | 43(22) | TCTACTGACCCTACTGTG |
| | 35(9) | CTGCTGTGTCTTCTAGTGA | | 44(23) | TACTAGTGAACAATATAAGCA |
| | 39(10) | ATAGAGTCTTCCATACCTTC | | 70(24) | GAAACGGCCATACCTG |
| | 45(11) | TAATTTAACATTATGTGCCTC | | 72(25) | GCGTCCTCTGTATCAGA |
| | 51(12) | TGCTGCGGTTTCCCCAA | | 81(26) | AGCTACATCTGCTGCTGCAGA |
| | 52(13) | GAATACCTTCGTCATGGC | | 81(27) | AACACCGAATCAGAATATAAACCTACC |
| | 53(14) | AACCACACAGTCTATGTCTACA | | 87(28) | AACCACTGAATATGACCCCACA |
| | 56(15) | CAGAACAGTTAAGTAAATATG | | | |
| | 58(16) | TATGCACTGAAGTAACTAAG | | | |
| | 59(17) | TCTACTACTTCTTCTATTCC | | | |
| | 68(18) | CAGACTCTACTGTACCAGC | | | |
| HPV-Universal | | GNCATGNNGARGAATNTGA (Seq. No. 29) | | | |

### Example 5: Luminous and Color Test of Reverse Blot Hybridization REBA

In order to perform a reverse blot hybridization reaction, MY11/09-GP5-1/GP6+ primer marked with 5'-biotin (Biotin) was prepared in order to obtain PCR product having a base sequence that can complementary-bind to the probe. 20 µl of the 150 bp PCR product obtained with the primer was hybridized to the membrane attached with HPV specific probes using a miniblotter. That is, 15 µl of the PCR product that was confirmed to be amplified was mixed with the same amount of DS (0.2 N NaOH and 0.2 mM EDTA), and then left at room temperature for 5 minutes to make a single strand; and then diluted with 115 µl 2X SSPE/0.1% (w/v) SDS. Before hybridizing the PCR product to the membrane, the PCR product was added in 100 ml 2X SSPE/0.1% (w/v) SDS solution and then reacted at room temperature for 5 minutes to activate the membrane; and then the membrane was put on a support cushion that was put on the miniblotter. Extra humidity in a slot was removed by using a suction apparatus and then the PCR product was deposited to be perpendicular to the direction that attaches the probes. The direction that attaches oligonucleotide probe was marked on the membrane with an ink before hybridizing. Empty slots around the solts deposited with the PCR product were filled with 2X SSPE/0.1% (w/v) SDS to prevent cross flow. The hybridization forming reaction was performed at 50°C for 30 minutes on even ground. Since then, the remaining sample was completely removed from the miniblotter with the suction apparatus, and then the membrane was twice washed with 100 ml 2X SSPE/0.5% SDS solution at 50°C for 10 minutes.

The membrane was put in a rolling bottle; well mixed with streptavidine-alkaline phosphatase conjugate (AP conjugate, Roche Applied Science, Germany) that was diluted with a suitable amount of 2X SSPE/0.5% SDS to be 1:2000 (v/v); and then reacted at room temperature for 30 minutes. Since then, the membrane was twice washed with 100 ml 2X SSPE/0.5% SDS solution at room temperature and then twice washed with 100 ml 2X SSPE solution. In order to detect a chemical fluorescence, the membrane was put in 10 ml CDP-Star™ detection reagent (Amersham pharmacia biotech., Buckinghamshire, England) to react for 4 minutes, and then exposed to Hyper sensitivity film for 20 minutes to confirm the result (FIG. 1).

For a color reaction, the membrane after completing the AP conjugate reaction was washed with TBS (50 mM Tris-HCl, 150 mM NaCl, pH 7.5) twice a minute; and then reacted with a substrate solution for a color formation, i.e., NBT/BClP [Nitro Blue Tertrazolium Chloride and 5-Bromo-4-Chloro-3-indolyl Phosphate, Toluidine salt in 67% DMSO (v/v), Roche Applied Science, Germany], that was diluted with TBS (100 mM Tis-HCl, 100 mM NaCl, 50 mM MgCl₂, pH 9.5) to be 1:50 (v/v), for 5 minutes at room temperature. A desired color formation was completed; then the solution for the color formation was completely removed; and then the reaction was stopped by adding tertiary distilled water.

### Example 6: Plasmid DNA Cloning of Clinical Specimen

In order to finally verify HPV genotype that was not held as a clone, HPV genotype 42 and 45 obtained from Biometrics, and the specimen that was subjected to be typing through a reverse blot hybridization method, they were performed with PCR using MY11/09-GP5-1/6 primer, and then the cloning was performed by using TOPO TA Cloning kit. A positive clone was analyzed by asking sequencing to Cosmo to analyze whether the genotype was correct, and then a final HPV type was obtained.

### Example 7: Comparison of Sensitivity with DNA Chip

HPV clinical samples were supplied from Chonnam national university medical school (Gwangju, Korea) and all of HPV testing was performed by using DNA chip.

Meanwhile, HPV gene-HPV extraction, PCR, and a reverse blot hybridization analysis were performed by using the similar method to Examples of the present invention as mentioned above in order to compare sensitivity with DNA chip.

### Example 8: Comparison of Conventional Two-tube PCR and One-tube PCR According to Present Invention

Two-tube nested PCR to HPV was disclosed in Example 3, and the like of Korean Patent Publication No. 10-2009-0129639 in detail. In short, first PCR was performed by using outer primer (MY11/09 primer) in 45 cycles, and then the resulting product was subjected to be PCR using inner primer (GP5/6 primer) in 45 cycles again. At this time, it has been confirmed that the sensitivity of PCR was 100 ag (FIG. 10). Since when the resulting product was subjected to be second amplication after performing the first polymerase chain reaction, the new reagents were added again, the two-tube nested PCR has an advantage in that the reaction could be smoothly performed, and since one pair of primers was used for a relatively little number of the reaction, the two-tube nested PCR has advantages in that it can increase excellent sensitivity and specificity. However, two amplication processes were performed by exchanging tube so that the two-tube nested PCR has a risk in that a contamination was often occurred.

In order to solve the above problems, the present invention established the one-tube nested PCR condition that can decrease the problem of the contamination and also can not affect the sensitivity of the conventional two-tube PCR (FIG. 11) at the same time by adding two pairs of primers (MY11/9-GP5-1/6) in one tube together, first amplifying at 55°C in 15 cycles, and then performing the nested PCR at decreased temperature (52°C), as Example 3 as mentioned above.

Hereinafter, the results of the Examples will be disclosed.

### 1) HPV Typing of ATCC Clone (FIG. 2)

From the results of performing PCT with the clones obtained from ATCC, it has been verified that all of PCR products were amplified, excepting HPV type 43 and the genotypes of the products could be re-verified through reverse blot hybridization to HPV specific probe of the present invention. However, HPV type 43 was non-specifically bond. The experiment each independently used the product that was subjected to be PCR by extracting two plasmids. However, in the case of type 44, the result was obtained by extracting only one plasmid due to a problem in terms of the experiment.

### 2) Verification of Digene Positive Specimen and Genotype-confirmed Clinical Specimen via Sequencing through Reverse Blot Hybridization Method

In the case of the specimen that was confirmed as a high risk group from Digene (by HC2) positive specimens in MizMedi hospital, it has been confirmed that there were amplified PCR product of 450 bp and non-amplified PCR product obtained from first PCR using MY11/09 primer. However, it has been confirmed from the result of performing PCR using GP5-1/6+, i.e., nested primer with all of first PCR products that 31 specimens of total 44 specimens exhibited PCR positive; and it has been also confirmed from the result of performing the reverse blot hybridization with the 31 specimens (REBA, FIG. 3) that 26 specimens were HPV type. At this time, HPV type 56 clone obtained from ATCC was used as a positive control.

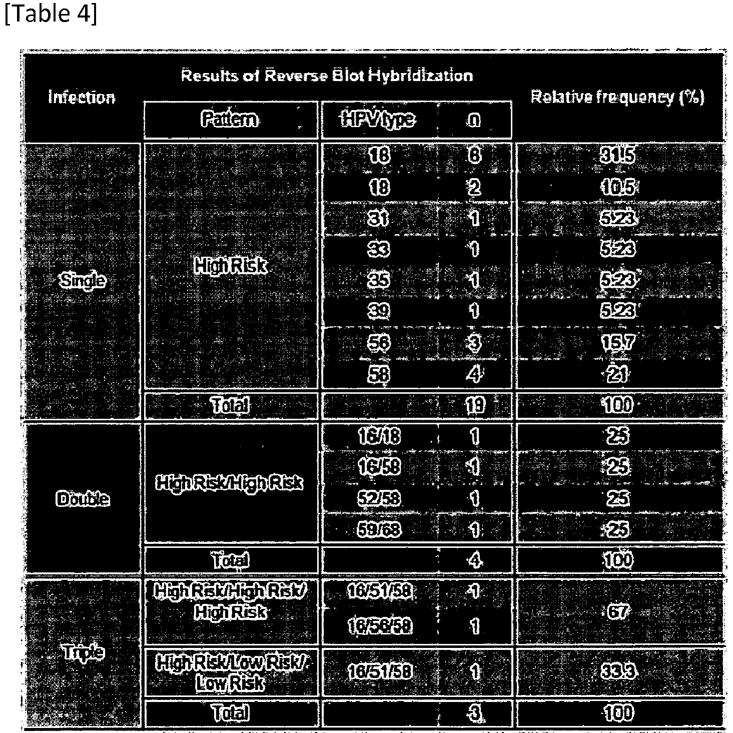

In terms of the distribution of HPV types, among total 19 in the case of a single infection, HPV type 16 is 31.5% and HPV type 58 is 21%. Overall genotype results of other clinical specimens are shown in Table 4. Especially, 7 specimens among 26 specimens, of which types were confirmed through reverse blot hybridization (REBA), were estimated to be super-infected and also all of them was estimated to be super-infected with high risk groups that were highly possible to have uterine cervical cancer (Table 4). In addition, a color test was performed with some clinical specimens (FIG. 4). Some samples among the some clinical specimens were confirmed by performing sequencing and the results were the similar (FIGS. 5 to 7).

**[Table 5]**

| **No.** | **REBA** Result | **Sequencing** Result | Similarity |
|---|---|---|---|
| **72** | **59** | **59** | **86%** |
| **73** | **58** | **58** | **89%** |
| **74** | **16, 53, 59** | **16,54** | **71%** |
| **75** | **56** | **56** | **94%** |
| **76** | **16** | **16** | **100%** |
| **78** | **16, 53, 56** | **56** | **87%** |
| **81** | **18** | **18** | **98%** |
| **82** | **31,53** | **31** | **77%** |
| **84** | **53** | **53** | **91%** |
| **88** | **58** | **58** | **97%** |
| **92** | **58** | **58** | **95%** |
| **102** | **16, 53, 56** | **16** | **94%** |
| **104** | **16, 70** | **16** | **92%** |
| **113** | **HPV (+)** | **62** | **94%** |
| **116** | **70, 58** | **70** | **97%** |
| **119** | **81** | **81** | **95%** |
| **132** | **58** | **58** | **97%** |

Table 5 is a result comparing REBA result and sequencing result in clinical specimens.

### 3) Obtainment of Plasmid DNA Clone

In order to finally performing genotyping with the specimens as mentioned above, the product of HPV L1 gene that was amplified with MY11/09-GP5-1/6 primer was obtained as a plasmid DNA clone. HPV type 31, 33, 35, 39, 45, 51, 52, 53, 56, 58, 66, 59, 68, 42, 43, 70, 72, 81, 84, and 87 clones were obtained from DNA isolated from the clinical specimens. The REBA results of the plasmid DNA clones were the similar (FIG. 8) as the result of other groups through an experimental method (REBA) with the probe used for the present invention as shown in FIG. 4. In addition, the results after a color reaction through the results obtained from the luminous experiment were the similar (FIG. 9).

### 4) Comparison of Sensitivity with DNA Chip

As shown in the following Table 6, it has been confirmed from the result of comparing sensitivity of DNA chip and the method of the present invention that DNA chip exhibits approximately 94% sensitivity and the present invention exhibits 100% sensitivity. In addition, it has been confirmed from the result of comparing the sequencing that REBA result is more precise. That is, the following Table 6 shows that 4 HPV (-) samples are in DNA chip (In other words, 4 samples are unreadable), but in the result of REBA, they are read as HPV positive sample attached to HPV universal probe. From the result of sequencing them, it has been confirmed that other type outside of 25 types presented in REBA are exhibited. Therefore, it has been confirmed that all of 70 were read through REBA method, but only 66 (94.29%) were read through DNA chip so that REBA method exhibits good result as compared with DNA chip.

In addition, in the case of using DNA chip, a high-degree of technology, a high-price device, such as a scanner, a calibration device, and the like, are required, but the REBA method according to the present invention does not require a special device for performing the test so that it can have an excellent result with a low cost.

**[Table 6]**

| **Genotyping results** | **DNA chip** | **M&D REBA HPV-ID^{®}** |
|---|---|---|
| **HPV (+)** | **62** | **63** |
| **Other types** | **4** | **7** |
| **HPV (-)** | **4** | **0** |
| **Total** | **70** | **70** |
| **Sensitivity** | **94.29%** | **100.00%** |
| **Concordance to DNA chip** | | **79%(55/70)** |
| **Concordance with sequencing analysis** | | **93%(65/70)** |

## Claims

1. An oligonucleotide probe composition for diagnosing HPV, comprising base sequences of Sequence Nos. 5 to 28.

2. A kit for diagnosing HPV, comprising the probe composition of claim 1 and a primer.

3. The kit of claim 2, wherein the primer is disclosed in Sequence Nos. 1 to 4.

4. The kit of claim 2 or 3, further comprising a marker for detecting PCR product.

5. A method of confirming HPV genotype, comprising:
a) attaching the probe of claim 1 to a solid support;
b) hybridizing HPV PCR product amplified to the probe; and
c) detecting the hybridized product.

6. The method of claim 5, wherein the solid support is a membrane, a slide, or a well plate.

7. The method of claim 5, wherein the PCR product is amplified by using the primer as disclosed in Sequence Nos. 1 to 4.

8. The method of claim 5, wherein the step of detecting is performed by using a luminous or color reaction.
